# EUROPÄISCHE PATENTSCHRIFT

(11) **EP 0 951 263 B1**
(45) Veröffentlichungstag und Bekanntmachung des Hinweises auf die Patenterteilung: **09.01.2002**
(21) Anmeldenummer: 97951262.1
(22) Anmeldetag: 21.11.1997
(51) Int. Cl.: A61F 13/02

(54) **APPLIKATIONSHILFE FÜR FOLIENVERBÄNDE**
APPLICATION AID FOR FILM DRESSINGS
AUXILIAIRE D'APPLICATION POUR PANSEMENT SOUS FORME DE FILM

(30) Priorität: 11.12.1996 DE 29621366 U
(43) Veröffentlichungstag der Anmeldung: 27.10.1999
(73) Patentinhaber: Lohmann GmbH & Co. KG, 56567 Neuwied (DE)
(72) Erfinder: ANHÄUSER, Dieter, D-56581 Melsbach (DE); ECKER, Jürgen, D-56567 Neuwied (DE); SCHENTEK, Heike, D-56581 Kurtscheid (DE)
(74) Vertreter: Flaccus, Rolf-Dieter, Dr.
(86) Internationale Anmeldenummer: EP9706528
(87) Internationale Veröffentlichungsnummer: WO9825559

(56) Entgegenhaltungen:
- EP-A- 0 120 570
- EP-A- 0 254 493
- EP-A- 0 308 122
- WO-A-89/07922
- GB-A- 2 297 260
- US-A- 4 915 102

## Beschreibung

Die Erfindung betrifft einen Folienverband, insbesondere für den menschlichen oder tierischen Körper, aus einer dünnen Folie, die mittels einer Haftklebeschicht auf dem Körper befestigt werden kann. Der Verband kann okklusiv oder nichtokklusiv sein.

Haftklebende, nichtokklusive Folienverbände werden seit längerem im medizinischen Bereich eingesetzt. Der Ausdruck "nichtokklusiv" bedeutet dabei, daß der Verband zwar für Bakterien und flüssiges Wasser undurchlässig ist, aber den Durchgang von Sauerstoff und Wasserdampf erlaubt. Besonders die letzte Eigenschaft gestattet, daß auf der Haut entstehender Wasserdampf durch den Verband entweichen kann. Die Verbände werden z. B. zur Abdeckung von Wunden und Verbrennungen, als Inzisionsfolien, zur dermalen Applikation von Wirkstoffen oder auch zur Fixierung von medizinischen Instrumenten wie Kathedern oder Kanülen auf der Haut eingesetzt.

Die in den Verbänden eingesetzten transparenten oder auch opaken Polymerfolien sind kontinuierlich, d. h. sie sind nicht perforiert und nicht mikroporös. Auch die aufgebrachte Haftklebeschicht muß in einer Weise wasserdampfdurchlässig sein, daß die Gesamtdurchlässigkeit des Verbandes nicht das erforderliche Maß unterschreitet. Um diese Forderungen zu erfüllen und einen flexiblen, anschmiegsamen Verband zur verfügung zu stellen, müssen die Folien extrem dünn sein. Allerdings führt dieser Umstand zu großen Schwierigkeiten bei der Applikation des Verbandes. So sind dazu oft zwei Personen erforderlich, um das Knittern der Folien und Aufsich-selbst-Haften der Klebeflächen zu vermeiden.

Okklusive Folienverbände, -Verbände mit einer sehr niedrigen oder keiner Wasserdampfdurchlässigkeit -, sind in bestimmten Fällen ebenfalls indiziert. Auch bei diesen Verbänden werden wegen der geforderten Anschmiegsamkeit extrem dünne Folien eingesetzt, so daß auch hier dieselben Applikationsschwierigkeiten wie bei den nichtokklusiven Verbänden auftreten.

Ein Beitrag zur Problemlösung wird in der DE-OS 19 35 916 offenbart, wonach die nicht mit Haftkleber beschichtete Fläche der Polymerfolie mit einer steifen Stützfolie abgedeckt wird, die erst nach Applikation des Verbandes abgezogen wird. Die verbleibenden Probleme liegen in der Handhabung des Verbandes bei der Applikation und dem Abziehen der Stützfolie, da dabei eine Kontamination der Haftklebeschicht unbedingt zu vermeiden ist.

Lösungsansätze liegen in der flächenmäßig größeren Ausgestaltung der Stützfolie gegenüber der Polymerfolie, wodurch haftkleberfreie Anfaßstellen ausbildbar sind (DE-PS 33 44 334), oder im Anbringen von Griffleisten an der Stützfolie in Randnähe (EP 0 066 899 und EP 0 473 918). Diese Vorschläge kranken daran, daß ihnen der Nachteil eines erheblichen Materialaufwandes verbunden mit teilweise aufwendigen Verfahrensschritten anhaftet.

In der US 4,915,102 wird eine weitere Möglichkeit beschrieben, bei der an einer Kante des Verbandes die einteilige Schutzschicht, d. h. wiederablösbare Schicht, die die Haftklebeschicht vor Applikation abdeckt, scharnierartig mit der Stützfolie verbunden ist. Bei der Applikation bleibt demnach die abgezogene Schutzschicht mit der Stützfolie verbunden und dient schließlich als Anfaßhilfe beim Abziehen der Stützfolie. An der Kante gegenüber der scharnierartigen Verbindung ist allerdings nur ein relativ schmaler, kleberfreier Anfaßrand vorgesehen, der aber zur Applikation gebraucht wird. Seine Erzeugung bedarf, wie oben schon erwähnt, besonderer technischer Verfahrensschritte.

In der EP 0 120 570 A1 wird ein Lösung für das Problem einer Kontamination der mit der Wunde in Berührung kommenden Fläche beim Abziehen der Schutzfolie vorgeschlagen, indem die Schutzfolie geteilt ist und von der Mitte her zu beiden Seiten gleichzeitig abgezogen werden kann. Die Schutzfolie wird dann von der aus Rückschicht und Kleber bestehenden Wundauflage abgetrennt. Dabei dienen sogenannte "release retarding means" dazu, ein vorzeitiges, unbeabsichtigtes Abreißen der Schutzfolie von dem Folienverband zu verhindern, indem dafür ein größerer Kraftaufwand notwendig ist als für das Abziehen der Schutzfolie von der Wundauflage.

Es ist daher Aufgabe der vorliegenden Erfindung, einen Folienverband zu schaffen, der bei möglichst einfacher Herstellbarkeit die bekannten Mängel in der Handhabbarkeit überwindet.

Die Lösung dieser Aufgabe wird dadurch erreicht, daß bei einem Folienverband aus einer flexiblen, anschmiegsamen Polymerfolie, die auf der hautabgewandten Seite mit einer Stützfolie lösbar verbunden und auf der hautzugewandten Seite mit einer haftklebenden Schicht ausgerüstet ist, die ihrerseits mit einer mindestens zweiteiligen abziehbaren Schutzschicht versehen ist, die Schutzschicht an zwei gegenüberliegenden Rändern scharnierartig mit der Stützfolie verbunden ist.

Diese Konstruktion erlaubt es unter anderem, die Stützfolie mit der Polymerfolie flächengleich zu gestalten und somit zusätzliche Schneid- und/oder Stanzvorgänge zu vermeiden. Die Schutzschichtteile lassen sich nach Ablösen von der Haftklebeschicht nach außen klappen und können zur Applikation des Verbandes angefaßt werden, ohne daß eine Kontamination der Haftklebeschicht in Kauf genommen werden muß. Nach Andrücken der Polymerfolie an Haut kann die Stützfolie mit den anhängenden Teilen der Schutzschicht bequem abgezogen werden.

Für die Realisierung der scharnierartigen Verbindung zwischen Schutzschicht und Stützfolie gibt es zahlreiche Möglichkeiten, von denen einige besonders bevorzugte beschrieben werden. Einmal kann die Schutzschicht streifenförmig den Rand des Verbandes überragen und ist nach Umknicken um diesen Rand auf der hautabgewandten Fläche der Stützfolie adhäsiv befestigt. Eine Paltlinie in der Schutzschicht begünstigt deren Beweglichkeit um die Scharnierachse. Die umgekehrte Konstruktion, d. h. überstehende Stützfolie ist nach Umknicken um den Rand auf der Unterseite der Schutzschicht gefestigt, ist prinzipiell auch möglich, wird aber wegen eventueller Nachteile auf Sonderfälle beschränkt bleiben.

In dem Falle, daß es die Umstände erlauben, die Stützfolie und die Schutzschicht aus demselben Material herzustellen, wird ein zusammenhängendes Flächengebilde um die Polymerfolie herum gefaltet, so daß die Enden auf die Seite der Klebeschicht zu liegen kommen. Die Adhäsion der Polymerfolie an diesem Flächengebilden wird nach bekannten Methoden geschaffen.

Bei einer weiteren bevorzugten Ausgestaltung der Erfindung haben Stützfotie, haftkleberbeschichtete Polymerfolie und Schutzschicht dieselben Außenkonturen und die scharnierartige Verbindung zwischen Stützfolie und Schutzschicht wird durch Anbringen eines längs geknickten Streifens gebildet, wobei ein Teil auf der hautabgewandten Seite der Stützfolie und der andere Teil auf der freien Seite der Schutzschicht haftet. Der Streifen ist vorzugsweise haftklebend ausgerüstet, kann aber auch durch andere geeignete Klebersysteme fixiert werden. Das Aufsiegeln bei Zimmertemperatur oder unter Wärmezufuhr bietet weitere Möglichkeiten der Fixierung. Selbstverständlich braucht der Streifen nicht die gesamte Länge der Scharnierkante zu umschließen, sondern es genügt in manchen Fällen, wenn nur Teile der Kante belegt sind. Als Material für den Streifen sind alle flexiblen Flächenmaterialien geeignet.

Vorzugsweise weist der Folienverband gemäß der Erfindung eine viereckige Kontur auf, wobei an gegenüberliegenden Kanten die scharnierartigen Verbindungen gebildet sind. Die Größe der Polymerfolie muß nicht der Größe der Stützfolie entsprechen; sie kann auch kleiner sein, so daß die Stützfolie die Polymerfolie überragt. Dabei kann die Kontur der Polymerfolie von der Stützfolie abweichen. Dies ist beispielsweise dann gegeben, wenn eine Polymerfolie mit abgerundeter Kontur von einer eckigen, - insbesondere viereckigen -, Stützfolie abgedeckt ist. Sind bei einer Polymerfolie mit abgerundeter Kontur keine geraden Kanten vorhanden, wie z. B. bei der Kreisform, wird beispielsweise eine Kontur der Stützfolie gewählt, die an gegenüberliegenden Seiten tangential zur abgerundeten Kontur der Polymerfolie so lange gerade Kanten aufweist, daß eine sichere Scharnierbildung gewährleistet ist.

Als Material für die Stützfolie kommen bekannte Polymere in Frage, wie z.B. Polyethylen, Polypropylen, Polyamid oder Polyester. Daneben sind aber auch textile Flächengebilde und Papier in Gebrauch. Die Schichtdecke der Stützfolie bewegt sich zwischen 20 µm und 200 µm, vorzugsweise 30- 80 µm. Die Stützfolie ist mit der Polymerfolie entweder mit einem geeigneten Kleber verklebt oder die Verbindung wird durch jene mechanischen Adhäsionskräfte bewerkstelligt, die entstehen, wenn die Polymerfolie durch Extrusion, Gießen oder eine andere bekannte Art der Folienherstellung unmittelbar auf der Stützfolie erzeugt wird.

Die Polymerfolie ist opak oder transparent und kann nach bekannten Techniken aus bekannten Rohstoffen hergestellt werden. Solche Rohstoffe sind z. b. Polyurethane, Polyvinylchloride, Polyvinylidenchloride, Polyvinylalkohole, Polyacrylate, Polysulfone, Polystyrole, Polypropylen, Polyamide, Ethylen-Vinylacetat-Copolymere, Polyester, Polycarbonate, Polyvinylfluorid oder andere fluorhaltige Polymere.

Für die Ausbildung einer nichtokklusiven Polymerfolie sind Polyurethane und für eine okklusive Polymerfolie Polyvinylidenchlorid bevorzugt. Die Schichtdecken geeigneter Polymerfolien liegen im Bereich von 7 µm - 12o µm, bevorzugt aber von 15 - 50 µm. Im Falle der nichtokklusiven Polymerfolien soll die Wasserdampfdurchlässigkeit mindestens 300 g x m⁻² x 24 h⁻¹ betragen.

Für den Aufbau der Haftklebeschicht sind die bekannten physiologisch unbedenklichen Haftklebematerialien geeignet. Als Beispiele angeführt seien Kautschuk, kautschukähnliche synthetische Homo-, Co- oder Blockpolymere, Polyacrylate und deren Copolymerisate, Polyurethane und Silikone. Der Flächenauftrag des Haftklebers liegt zwischen 15 - 80 g/m², vorzugsweise 30 - 50 g/m².

Das Material für die Schutzschicht kann jenes sein, das auch für die Stützschicht Verwendung findet. Darüberhinaus können aber auch beispielsweise Polytetrafluorethylen, Cellophan, Polyvinylchlorid, dehäsiv behandelte Papiere, Metallfolien und mit Polymeren überzogene Metallfolien eingesetzt werden. Die Flächengewichte liegen bei 30 - 250 g/m², vorzugsweise 50 - 150 g/m². Die mit der Haftklebeschicht in Kontakt befindliche Seite der Schutzschicht muß eine Wiederablösung mit einer Kraft erlauben, die kleiner ist als jene zur Ablösung der Stützfolie von der Polymerfolie. Die Enden der mindestens zweiteiligen Schutzschicht, die auf die Haftklebeschicht zu liegen kommen, sind vorteilhaft in gewohnter Weise mit Anfaßhilfen ausgestattet.

Die haftklebende Schicht kann Wirkstoffe enthalten und damit deren dermale Applikation erlauben. Besondere Anwendung findet daher die vorliegende Erfindung als transdermales therapeutisches System, das durch eine extrem hohe Anpassungsfähigkeit an die Haut, verbunden mit hervorragendem Tragekomfort, gekennzeichnet ist. Selbstverständlich kann wirkstoffhaltiges Material auch auf einem Teil der haftklebenden Schicht auf der hautzugewandten Seite angebracht sein.

Weiterhin können auf einem Teil der haftklebenden Schicht auf der hautzugewandten Seite flüssigkeitsabsorbierende Flächengebilde aufgebracht sein, so daß der Verband zur Wundversorung eingesetzt werden kann.

Die Erfindung wird anhand von Figuren beispielhaft weiter erläutert. Sie sind nicht maßstabsgetreu dargestellt und die Dicke der vorkommenden Schichten ist zum besseren Verständnis übertrieben stark wiedergegeben. Gleiche Elemente tragen dieselben Bezugszeichen.

Es zeigen:
- Fig. 1: eine perspektivische Darstellung eines Folienverbandes gemäß der Erfindung,
- Fig. 2 und 3: je ein Querschnittsfragment mit verschiedenen Scharnierausführungen,
- Fig. 4: eine perspektivische Darstellung eines Folienverbandes, bei dem die Stützfolie größer ist als die Polymerfolie, und
- Fig. 5: eine Aufsicht auf die Schutzschichtseite eines Folienverbandes mit runder Kontur

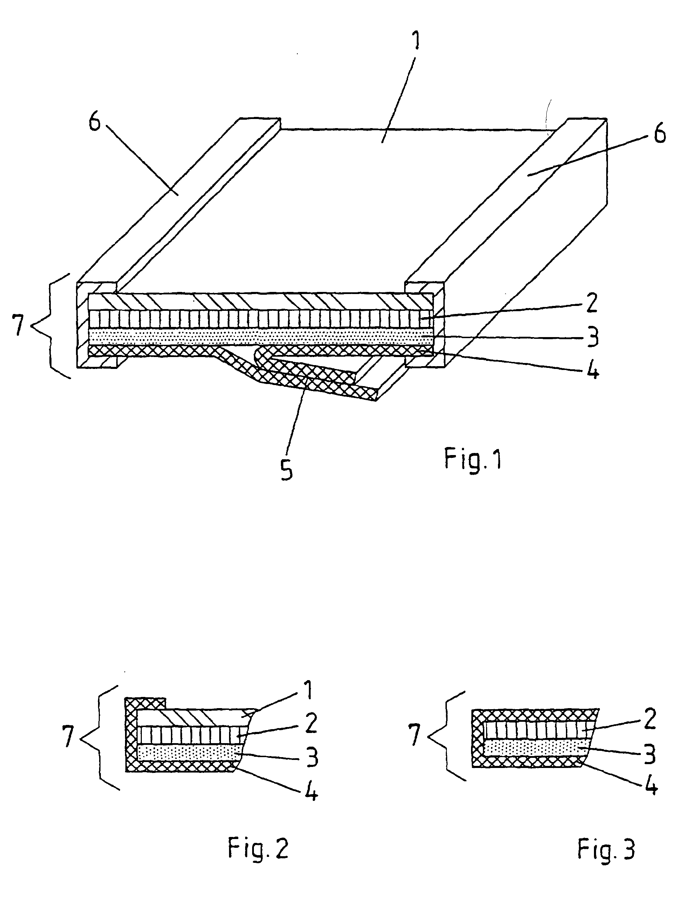

Fig. 1 zeigt in perspektivischer Darstellung den Aufbau eines erfindungsgemäßen Folienverbandes. Die flexible, anschmiegsame Polymerfolie 2 ist auf der hautabgewandten Seite von der gleich großen Stützfolie 1 abgedeckt. Auf der hautzugewandten Seite der Polymerfolie 2 ist eine Haftkleberschicht 3 angeordnet, die mit einer zweiteiligen Schutzschicht 4 versehen ist, wobei beide Teile durch überstehende Flächenteile mit Anfaßhilfen 5 ausgestattet sind. Auf zwei gegenüberliegende Kanten des Folienverbandes ist jeweils ein längs geknickter Streifen 6 aufgesetzt, der eine scharnieratige Verbindung zwischen 1 und 4 bildet.

Weitere Möglichkeiten der Scharnierbildung 7 sind in den Fig. 2 und 3 wiedergegeben. An den Fragmenten von Querschnitten ist zu erkennen, daß in Fig. 2 die Schutzschicht selbst das Scharniermaterial bildet und nach Umschlingung der Kante auf der Oberseite der Stützfolie 1 befestigt ist. In Fig. 3 sind Schutzschicht 4 und Stützfolie aus einem einheitlichen Material und erlauben es daher, durch einfache Faltung über die Kanten die gewünschte scharnierartige Verbindung zwischen den Außenschichten des Verbandes herzustellen.

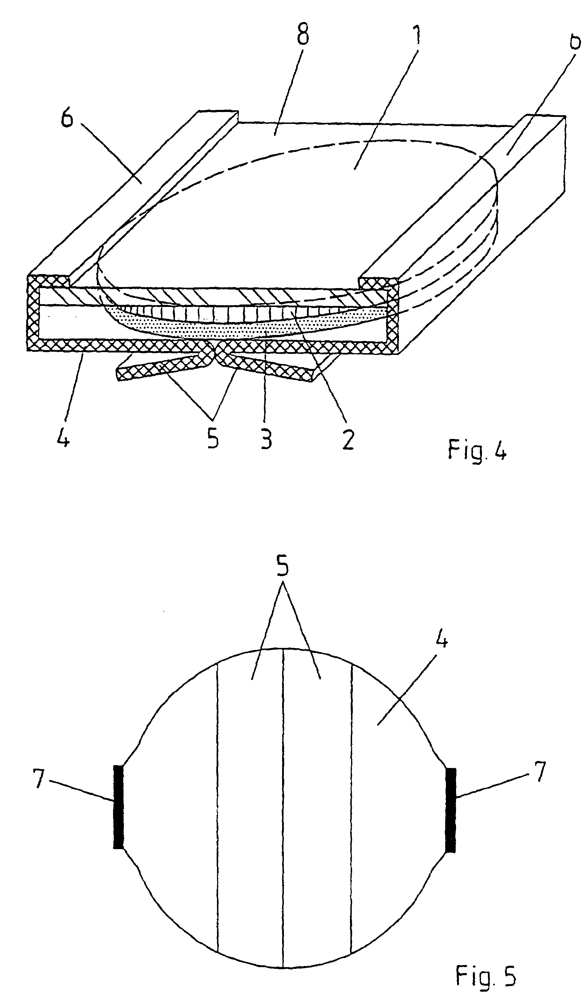

Fig. 4 dokumentiert in perspektivischer Darstellung, daß die flächenmäßige Ausdehnung der Stützfolie 1 größer sein kann als die der Polymerfolie 2, deren Kontur 8 gestrichelt angegeben ist. Des weiteren liegt hiermit ein Beispiel für Folienverbände mit abgerundeten Konturen vor. Die Ausgestaltung der Anfaßhilfen 5 ist hier in etwas anderer Weise gelöst ist als in Fig. 1.

Ein kreisrunder Folienverband mit fast flächengleichen Schichten erlaubt die Scharnierbildung durch gerade Kantenabschnittbildung an gegenüberliegenden Seiten, wie die Aufsicht auf die Schutzschichtseite 4 in Fig. 5 zeigt. Als weitere Merkmale sind die Anfaßhilfen zu erkennen. Diese Erfindungsvariante ermöglicht damit unter anderem die problemlose Applikation von wirkstoffhaltigen Pflastern mit äußerst dünner und anschiegsamer Trägerfolie fast beliebiger Kontur.

## Patentansprüche

1. Folienverband, insbesondere für den menschlichen oder tierischen Körper, aus einer flexiblen, anschmiegsamen Polymerfolie (2), die auf der hautabgewandten Seite mit einer Stützfolie (1) lösbar verbunden und auf der hautzugewandten Seite mit einer haftklebenden Schicht (3) ausgerüstet ist, die ihrerseits mit einer mindestens zweiteiligen abziehbaren Schutzschicht (4) verse hen ist, **dadurch gekennzeichnet, daß** die Schutzschicht (4) an zwei gegenüberliegenden Rändern scharnierartig (7) mit der Stützfolie (1) verbunden ist.

2. Folienverband nach Anspruch 1, **dadurch gekennzeichnet, daß** die scharnierartige Verbindung (7) zwischen Schutz schicht 4) und Stützfolie (1) durch adhäsives Befestigen eines streifenförmigen, den Rest des Verbandes überragenden Teiles der Schutzschicht entlang einer Faltlinie auf der hautabgewandten Fläche der Stützfolie (1) gebildet ist.

3. Folienverband nach Anspruch 1, **dadurch gekennzeichnet, daß** die Schutzschicht (4) und die Stützfolie (1) aus demselben Material bestehen und zusammenhängend über zwei gegenüberliegende Ränder des Verbandes unter Ausbildung eines Scharnieres (7) gefaltet sind, wobei die Enden des Flächengebildes auf die Seite der Klebeschicht (3) zu liegen kommen.

4. Folienverband nach Anspruch 1, **dadurch gekennzeichnet, daß** die scharnierartige Verbindung (7) zwischen Schutzschicht (4) und Stützfolie (1) durch einen aufgesetzten, längs geknickten Streifen (6) gebildet ist.

5. Folienverband nach Anspruch 4, **dadurch gekennzeichnet, daß** der Streifen (6) aufgeklebt ist.

6. Folienverband nach Anspruch 4, **dadurch gekennzeichnet, daß** der Streifen (6) aufgesiegelt ist.

7. Folienverband nach Ansprüchen 4 -6 , **dadurch gekennzeichnet, daß** der Streifen (6) nur Teile der Scharnierkante umschließt.

8. Folienverband nach einem oder mehreren der vorangehenden Ansprüche, **dadurch gekennzeichnet, daß** die flexible, anschmiegsame Polymerfolie (2) eine abgerundete Kontur aufweist.

9. Folienverband nach einem oder mehreren der vorangehen den Ansprüche, **dadurch gekennzeichnet, daß** die flexible, anschmiegsame Polymerfolie (2) kleiner dimensioniert ist als die Stützfolie (1).

10. Folienverband nach einem oder mehreren der vorgenannten Ansprüche, **dadurch gekennzeichnet, daß** die haftklebende Schicht (3) wirkstoffhaltig ist.

11. Folienverband nach einem oder mehreren der vorangehen den Ansprüche, **dadurch gekennzeichnet, daß** auf einem Teil der haftklebenden Schicht (3) auf der hautzugewandten Seite wirkstoffhaltiges Material angebracht ist.

12. Folienverband nach einem oder mehreren der vorangehenden Ansprüche, **dadurch gekennzeichnet, daß** auf einem Teil der haftklebenden Schicht (3) auf der hautzugewandten Seite flüssigkeitsabsorbierendes Flächengebilde angebracht ist.

## Claims

1. Film dressing, in particular for the human or animal body, consisting of a flexible, conformable polymer film (2) which is detachably bonded on the side facing away from the skin to a supporting sheet (1) and is provided on the side facing the skin with a pressure sensitive adhesive layer (3) which is in turn provided with an at least two-part protecting layer (4) which can be pulled off, **characterized in that** the protecting layer (4) has hinge-like connections (7) at two opposite edges to the supporting sheet (1).

2. Film dressing according to Claim 1, **characterized in that** the hinge-like connection (7) between protecting layer (4) and supporting sheet (1) is formed by adhesive fastening of a strip-like part, which projects beyond the remainder of the dressing, of the protecting layer'along a fold line on the surface of the supporting sheet (1) facing away from the skin.

3. Film dressing according to Claim 1, **characterized in that** the protecting layer (4) and the supporting sheet (1) consist of the same material and are folded continuously over two opposite edges of the dressing to form a hinge (7) with the ends of the flat material coming to rest on the side of the adhesive layer (3).

4. Film dressing according to Claim 1, **characterized in that** the hinge-like connection (7) between the protecting layer (4) and supporting sheet (1) is formed by an attached, longitudinally creased strip (6).

5. Film dressing according to Claim 4, **characterized in that** the strip (6) is bonded on.

6. Film dressing according to Claim 4, **characterized in that** the strip (6) is sealed on.

7. Film dressing according to Claims 4-6, **characterized in that** the strip (6) encloses only parts of the edge of the hinge.

8. Film dressing according to one of more of the preceding claims, **characterized in that** the flexible, conformable polymer film (2) has a rounded contour.

9. Film dressing according to one or more of the preceding claims, **characterized in that** the flexible, conformable polymer film (2) has smaller dimensions than the supporting sheet (1).

10. Film dressing according to one or more of the abovementioned claims, **characterized in that** the pressure sensitive adhesive layer (3) contains active substances.

11. Film dressing according to one or more of the preceding claims, **characterized in that** active substance-containing material is applied to part of the pressure sensitive adhesive layer (3) on the side facing the skin.

12. Film dressing according to one or more of the preceding claims, **characterized in that** liquid-absorbing flat material is applied to part of the pressure sensitive adhesive layer (3) on the side facing the skin.

## Revendications

1. Pansement sous forme de film, en particulier pour le corps humain ou de l'animal, constitué d'un film polymère (2) flexible et souple sur lequel est appliqué, du côté opposé à la peau, un film de maintien (1) dont il peut être détaché, et qui est pourvu, du côté tourné vers la peau, d'une couche adhésive (3) ayant, quant à elle, une couche protectrice (4) enlevable composée d'au moins deux parties, **caractérisé en ce que** la couche protectrice (4) et le film de maintien (1) sont tenus ensemble sur deux bords opposés par un assemblage en forme de charnière (7).

2. Pansement sous forme de film selon la revendication 1, **caractérisé en ce que** l'assemblage en forme de charnière (7) entre la couche protectrice (4) et le film de maintien (1) est formé par une partie de la couche protectrice qui, en forme de bande dépassant le reste du pansement, est fixée par adhésion, le long d'une ligne de pliage, sur la surface du film de maintien (1) opposée à la peau.

3. Pansement sous forme de film selon la revendication 1, **caractérisé en ce que** la couche protectrice (4) et le film de maintien (1) sont constitués de la même matière et sont pliés, tout en se prolongeant réciproquement, sur deux bords opposés du pansement de manière à former une charnière (7), les extrémités de cette surface venant s'appliquer sur la face de la couche adhésive (3).

4. Pansement sous forme de film selon la revendication 1, **caractérisé en ce que** l'assemblage en forme de charnière (7) entre la couche protectrice (4) et le film de maintien (1) est formé par la pose d'une bande (6) pliée dans le sens de la longueur.

5. Pansement sous forme de film selon la revendication 4, **caractérisé en ce que** la bande (6) est collée.

6. Pansement sous forme de film selon la revendication 4, **caractérisé en ce que** la bande (6) est scellée.

7. Pansement sous forme de film selon les revendications 4 à 6, **caractérisé en ce que** la bande (6) n'enveloppe que des parties de l'arête de la charnière.

8. Pansement sous forme de film selon une ou plusieurs des revendications précédentes, **caractérisé en ce que** le film polymère (2) flexible et souple présente un contour arrondi.

9. Pansement sous forme de film selon une ou plusieurs des revendications précédentes, **caractérisé en ce que** les dimensions du film polymère (2) flexible et souple sont inférieures à celles du film de maintien (1).

10. Pansement sous forme de film selon une ou plusieurs des revendications précédentes, **caractérisé en ce que** la couche adhésive (3) contient des substances actives.

11. Pansement sous forme de film selon une ou plusieurs des revendications précédentes, **caractérisé en ce qu'**une matière contenant des substances actives est appliquée sur une partie de la face de la couche adhésive (3) tournée vers la peau.

12. Pansement sous forme de film selon une ou plusieurs des revendications précédentes, **caractérisé en ce qu'**une surface absorbant des liquides est appliquée sur une partie de la face de la couche adhésive (3) tournée vers la peau.
